# FASCICULE DE BREVET EUROPEEN

(11) **EP 0 778 894 B1**
(45) Date de publication et mention de la délivrance du brevet: **19.04.2006**
(21) Numéro de dépôt: 95929143.6
(22) Date de dépôt: 28.08.1995
(51) Int. Cl.: C12N 15/50, C07K 14/165, A61K 39/215

(54) **VACCIN DE LA PERITONITE INFECTIEUSE FELINE**
IMPFSTOFF FÜR DIE INFEKTIÖSE PERITONITIS DER KATZEN
INFECTIOUS PERITONITIS VACCINE

(30) Priorité: 29.08.1994 FR 9410379
(43) Date de publication de la demande: 18.06.1997
(73) Titulaire: MERIAL, 69002 Lyon (FR)
(72) Inventeur: DARTEIL, Rapha¬l, F-69007 Lyon (FR); CORAPI, Wayne, Staten Island, NY 10312 (US); AUDONNET, Jean-Chirstophe, F-69006 Lyon (FR); CHAPPUIS, Gilles-Emile, F-69006 Lyon (FR)
(74) Mandataire: Nargolwalla, Cyra
(86) Numéro de dépôt international: PCT/FR1995/001128
(87) Numéro de publication internationale: WO 1996/006934

(56) Documents cités:
- WO-A-93/23422
- WO-A-95/07987
- JOURNAL OF GENERAL VIROLOGY, vol. 74, READING GB, pages 745-749, OLSEN, C. ET AL. 'Identification of antigenic sites mediating antibody-dependent enhancement of feline infectious peritonitis virus infectivity' cité dans la demande
- JOURNAL OF VIROLOGY, vol. 66, no. 11, pages 6695-6705, CORAPI, W. ET AL. 'Monoclonal antibody analysis of neutralization and antibody-dependent enhancement of feline infectious peritonitis virus' cité dans la demande
- JOURNAL OF GENERAL VIROLOGY, vol. 68, no. 10, READING GB, pages 2639-2646, DE GROOT, R. ET AL. 'cDNA cloning and sequence analysis of the gene encoding the peplomer protein of feline infectious peritonitis virus' cité dans la demande
- JOURNAL OF VIROLOGY, vol. 69, no. 5, Mai 1995 pages 2858-2862, CORAPI, W. ET AL. 'Localization of antigenic sites of the S glycoprotein of feline infectious peritonitis virus involved in neutralization and antiboby-dependent enhancement'
- KIDA ET AL.: "Selection of antigenic variants of the S glycoprotein of feline infectious peritonitis virus and analysis of antigenic sites involved in neutralization" J.VET.MED.SCI., vol. 61, no. 8, 1999, pages 935-938,
- LAFON ET AL.: J.GEN.VIROL., vol. 71, 1990, pages 1689-1696,

## Description

### Domaine de l'invention

La présente invention a trait à des vaccins contre la Péritonite Infectieuse Féline (PIF) préparés à partir de la glycoprotéine SPIKE (S) du virus de la PIF dont les épitopes facilitants majeurs ont été modifiés par mutagénèse. Ces vaccins permettent une protection des chats vaccinés contre la PIF sans provoquer chez ces derniers le phénomène de facilitation qui conduit à une évolution accélérée de la maladie.

### Etat de l'art antérieur

Le virus de la Péritonite Infectieuse Féline (FIPV = Feline Infectious Peritonitis Virus) est un virus à ARN simple brin positif, enveloppé, qui, au sein de la famille des Coronaviridae, appartient au groupe antigénique qui comprend le coronavirus félin entérique (FECV), le coronavirus canin (CCV), le virus de la gastroentérite transmissible du porc (TGEV) et le coronavirus respiratoire porcin (PRCV) (Sanchez C. et al. Virology, 1990, 174, 410-417). Ce virus provoque une maladie complexe et toujours mortelle chez les chats, connue sous le nom de Péritonite Infectieuse Féline (PIF). Le virus de la PIF se singularise au sein des coronavirus parce qu'il induit chez le chat l'apparition d'anticorps facilitant l'infection par le virus et accélérant l'évolution de la maladie. Des chats possédant des anticorps neutralisants anti-FIPV suite à une infection naturelle antérieure par ce virus, suite à un transfert passif d'anticorps ou suite à une vaccination, développent très fréquemment une maladie beaucoup plus intense et beaucoup plus rapide que celle de chats simplement infectés pour la première fois en l'absence d'anticorps spécifiques (Pedersen N. et Boyle J., Am. J. Vet. Res. 1980, 41, 868-876 ; Weiss R. et al., Comp. Immunol. Microb. Infect. Dis. 1981, 4, 175-189 ; Weiss R. et al., Am. J. Vet. Res. 1980, 41, 663-671). On pense que la liaison des immuns complexes anticorps-virus aux récepteurs Fc présents à la surface des macrophages constitue le mécanisme qui facilite l'accélération de l'entrée du virus dans les cellules et sa diffusion rapide au sein de l'organisme (Porterfield, J. Advances in Virus research, 1986, 31, 335-355 ; Weiss et al. 1981). Ce phénomène de facilitation n'a été observé au sein des coronavirus qu'avec le virus de la PIF.

Le virus de la PIF comprend trois protéines structurales. La plus importante en taille est la protéine "SPIKE" ou spicule (S). Cette protéine S est fortement glycosylée et c'est elle qui induit chez le chat à la fois des anticorps neutralisants et des anticorps facilitants. Des études *in vitro* réalisées avec des anticorps monoclonaux neutralisants dirigés contre le virus de la PIF ont montré que les épitopes neutralisants majeurs sont tous situés sur la glycoprotéine S et qu'ils correspondent, à un large degré, aux épitopes impliqués dans le phénomène de facilitation (Corapi W. et al., J. Virol. 1992, 66, 6695-6705 ; Olsen C. et al., J. Virol. 1992, 66, 956-965).

Une vaccination efficace contre la PIF doit conduire à l'apparition d'anticorps neutralisants sans qu'il y ait induction d'anticorps facilitants. Un tel vaccin n'a jamais pu être mis au point jusqu'ici. Les vaccins recombinants qui ne contiennent pas la glycoprotéine S peuvent probablement fournir la meilleure alternative pour les vaccins PIF futurs, mais ces antigènes ne contribuent que partiellement à l'induction de la réponse neutralisante contre le virus PIF. Des trois antigènes viraux structuraux, seule la glycoprotéine S est capable d'induire une importante réponse neutralisante. Malheureusement, cette glycoprotéine induit aussi l'apparition concomitante d'anticorps facilitants. Malgré son importance dans l'induction d'une bonne réponse neutralisante (et donc dans la réponse protectrice), la glycoprotéine S naturelle semble jouer un rôle essentiel dans le phénomène de facilitation de la PIF et ne peut donc être utilisée pour l'instant pour la fabrication de vaccins répondant aux critères exposés plus haut.

La localisation et la caractérisation des épitopes présents sur S et en particulier ceux responsables de la neutralisation et de la facilitation est donc nécessaire pour déterminer les modifications à apporter à la glycoprotéine S (ou au gène qui code pour cette protéine) pour en faire un immunogène efficace pour la vaccination des chats contre la PIF.

La séquence nucléotidique et la séquence protéique de la glycoprotéine S du virus de la PIF ont été déterminées (de Groot R. et al. EP-A-0 264 979). Cette demande de brevet n'enseigne pas comment identifier les épitopes neutralisants et/ou les épitopes facilitants sur S. Ce document n'enseigne pas non plus comment utiliser la séquence de S pour fabriquer un vaccin efficace et non facilitant contre la PIF.

La demande de brevet PCT WO-A-93/23421 revendique l'utilisation d'une glycoprotéine S tronquée ou d'une séquence d'acide nucléique ne codant que pour une partie de S. En particulier, la région très conservée située à l'extrémité carboxy-terminale de S (124 derniers acides aminés) est revendiquée pour la préparation d'un vaccin "universel" contre les coronavirus. Ce document est très général et n'enseigne pas comment réaliser un vaccin PIF qui n'induise pas d'anticorps facilitants chez le chat. Il en va de même de la demande de brevet PCT WO-A-93/23 422 qui décrit des constructions mixtes de glycoprotéine S chimère FECV-FIPV incluant des fragments de FIPV S 542-597, 594-1454 ou 651-1454.

La demande de brevet PCT WO-A-92/08487 revendique l'utilisation de divers peptides sélectionnés sur les protéines S, ou codés par les gènes S, de diverses souches de virus FIPV ou par la séquence du gène S de FECV, à des fins de diagnostic, de traitement ou de prévention de la PIF chez le chat. En particulier, le peptide 598-615 de la séquence protéique de S du virus FIPV souche 79-1146 est revendiqué pour être utilisé sous la forme d'une protéine de fusion avec la galactokinase, protéine recombinante pouvant être utilisée ensuite pour le diagnostic des anticorps anti-PIF chez les chats infectés ou comme vaccin recombinant pour induire une protection contre la PIF chez les chats. Bien qu'envisageant des variations dans les séquences des peptides revendiqués, ce document n'enseigne pas précisément quels doivent être les changements dans les séquences proposées, et n'enseigne ni comment réaliser un vaccin PIF non facilitant, ni quelles sont les régions de la glycoprotéine S impliquées dans ce phénomène.

La demande de brevet GB-A-2 282 601, publiée après la date de priorité de la présente demande, propose de réaliser un vaccin à base d'une protéine S modifiée pour éviter la facilitation, par modification ou délétion de l'un au moins des sites antigéniques dénommés D (correspond aux acides aminés 496-524), A1 (correspond aux acides aminés 531-555) et A2 (correspond aux acides aminés 584-604), de manière à rendre ces sites antigéniquement inactifs.

Des efforts importants ont été réalisés pour identifier les sites antigéniques majeurs présents sur les protéines S du virus TGEV (Transmissible Gastro-Enteritis Virus) (Correa I. et al., J. Gen. Virol. 1990, 71, 271-279 ; Delmas B. et al., J. Gen. Virol. 1990, 71, 1313-1323), BCV (Bovine CoronaVirus) (Yoo D. et al., Virology 1991, 183, 91-98), MHV (Mouse Hepatitis Virus) (Takase-Yoden S. et al., Virus Res. 1990, 18, 99-108 ; Stuhler A. et al., J. Gen Virol. 1991, 72, 1655-1658) et FIPV (Corapi W. et al., J. Virol. 1992, 66, 6695-6705 ; Olsen C. et al., J. Virol. 1992, 66, 956-965 ; Olsen C. et al., J. Gen. Virol. 1993, 74, 745-749). Dans tous les cas, de multiples domaines neutralisants ont été identifiés, et les domaines immunodominants ont été généralement localisés sur la partie S1 de la protéine.

Les études portant spécifiquement sur le virus de la PIF ont montré l'existence sur la protéine S d'épitopes induisant à la fois une réponse neutralisante et une réponse facilitante vis-à-vis de l'infection avec FIPV (Corapi W. et al., J. Virol. 1992, 66, 6695-6705 ; Olsen C. et al . , J. Virol . 1992, 66, 956-965 ; Olsen C. et al . , J. Gen. Virol. 1993, 74, 745-749). Ces mêmes auteurs ont montré que les anticorps monoclonaux neutralisants et facilitants de spécificité anti-S pouvaient être répartis en 6 groupes principaux selon leur capacité à reconnaître différentes souches de virus PIF et différents mutants résistants à la neutralisation par ces monoclonaux (mutants *"mar"* (monoclonal antibody résistant). Toutefois, les épitopes correspondant aux régions antigéniques majeures sur FIPV S n'ont pas été caractérisés. Tous les anticorps monoclonaux non neutralisants décrits par ces auteurs (Olsen C. et al., J. Virol. 1992, 66, 956-965) sont également non facilitants dans un test de facilitation *in vitro*, ce qui renforce l'hypothèse d'une relation étroite entre neutralisation et facilitation dans le cas du virus de la PIF. La facilitation de l'infection virale par les anticorps survient lorsque les monocytes ou les macrophages sont infectés plus efficacement par les immuns complexes, par une endocytose dépendante de récepteurs spécifiques, que par le virus seul. Malgré toutes les études réalisées sur le phénomène de facilitation dépendant des anticorps, de nombreuses questions restent sans réponse. En particulier, on ne sait pas quels sont les composants viraux spécifiques responsables de la facilitation pour chaque virus. Les études réalisées jusqu'ici chez FIPV indiquent que la facilitation dépend essentiellement d'épitopes présents sur S (Olsen C. et al. 1993 ; Vennema H. et al., J. Virol. 1990, 64, 1407-1409).

Hohdatsu T. et al. (Arch. Virol. 1991, 120, 207-217) ont trouvé que des anticorps monoclonaux anti-FIPV M pouvaient induire une facilitation de l'infection *in vitro*. Ceci n'a pas été confirmé *in vivo* avec les études réalisées avec des recombinants vaccine/FIPV M et vaccine/FIPV N. L'immunisation de chats avec ces deux recombinants n'a pas permis d'observer une facilitation induite par l'une ou l'autre de ces deux protéines (Vennema H. et al. 1990). Si M et N jouent un rôle dans la facilitation, c'est certainement à un degré très inférieur à celui joué par S. Au cours des études réalisées avec les différents systèmes viraux où la facilitation peut être observée, un fait constant a été mis en évidence : des épitopes individuels sont capables d'induire à la fois des anticorps neutralisants et des anticorps facilitants. Ceci a été démontré pour FIPV (Corapi W. et al., J. Virol. 1992, 66, 6695-6705 ; Olsen C. et al., J. Virol. 1992, 66, 956-965 ; Hohdatsu T. et al., Arch. Virol. 1991, 120, 207-217), pour le virus de la dengue (Morens D. et Halstead S., J. Gen. Virol. 1990, 71, 2909-2917), et pour HIV (Robinson W. Jr., J. Virol. 1991, 65, 4169-4176).

Les résultats récents des tests réalisés avec des vaccins PIF expérimentaux semblent fournir l'argument le plus solide à ce jour pour l'existence d'une relation directe entre la facilitation observée *in vitro* et la maladie accélérée *in vivo* chez le chat. L'inoculation de chats avec des recombinants du virus de la vaccine exprimant la protéine S de la souche FIPV 79-1146 sensibilise les chats et induit après épreuve une maladie accélérée chez les chats vaccinés par rapport aux chats témoins non vaccinés (Vennema H. et al., J. Virol. 1990, 64, 1407-1409). L'inoculation de recombinants vaccine exprimant soit la protéine M, soit la protéine N, n'a pas prédisposé les chats à une maladie accélérée. Ces résultats *in vivo* sont à mettre en parallèle avec les résultats *in vitro* démontrant une localisation majoritaire des épitopes facilitants sur S (Corapi W. et al., J. Virol. 1992, 66, 6695-6705 ; Olsen C. et al., J. Virol. 1992, 66, 956-965). De plus, des expériences récentes réalisées pour étudier l'efficacité d'un autre candidat vaccin pour la PIF ont démontré une association statistiquement significative entre la capacité d'un sérum de chat à induire une facilitation *in vitro* et le développement chez le même chat d'une maladie accélérée (Olsen C., Vet. Microb. 1993, 36, 1-37).

### Description de l'invention

La présente invention a pour objet la caractérisation des épitopes impliqués dans la facilitation de l'infection par le virus de la PIF. La connaissance précise des structures moléculaires responsables du mécanisme de facilitation permet de concevoir des antigènes n'induisant pas l'apparition d'anticorps facilitants. Ces antigènes sont les composants essentiels d'un vaccin PIF efficace.

De manière surprenante, il a été découvert, par analyse de la séquence du gène S de virus FIPV mutants résistants à la neutralisation par des anticorps monoclonaux neutralisants et facilitants, ou résistants à des anticorps monoclonaux uniquement neutralisants et non facilitants, qu'il était possible de contourner le mécanisme d'induction de la facilitation par la glycoprotéine S. Deux sites antigéniques majeurs ont été caractérisés avec les anticorps monoclonaux étudiés : A1 et A2. Ces sites sont de manière surprenante situés tous les deux dans la même région de la protéine S. Il semble que les anticorps fortement neutralisants et facilitants reconnaissent les deux sites en même temps. Cette information suggère que la liaison simultanée des deux épitopes par un même anticorps joue un rôle direct dans la facilitation. En effet, parallèlement à cette première dévouverte, il a été découvert que les anticorps neutralisants, mais non facilitants, ne reconnaissent que le site A2. La facilitation serait donc due à une modification conformationnelle par rapprochement entre les deux épitopes A1 et A2. La région A2 inclut les acides aminés 637-662 sur la séquence protéique de S (De Groot R. et al., J. Gen. Virol. 1987, 68, 2639-2646). La nature hydrophile de cette région et le fait que les 3 anticorps monoclonaux testés reconnaissent tous ce petit domaine suggèrent que A2 est un épitope neutralisant dominant de la protéine S. Par ailleurs, l'étroite homologie mise en évidence entre le site A1 et une partie du sous-site Aa identifié sur la protéine S de TGEV (Gebauer F. et al., Virology 1991, 183, 225-238) suggère que A1 qui comprend les acides aminés 562-598 doit être également un important épitope neutralisant pour le virus FIPV.

Le rapprochement ou la liaison simultanée, par un même anticorps, des sites A1 et A2 est nécessaire pour induire la facilitation par l'intermédiaire des anticorps.

La présente invention a pour objet la modification par génie génétique de la séquence du gène FIPV S dans la région des sites A1 et/ou A2, en particulier pour modifier l'un au moins des deux sites, de préférence le site A1 de manière que la protéine exprimée présente un épitope modifié, de telle sorte qu'elle n'induise plus d'anticorps facilitants et/ou le site A2. La région A1 peut être modifiée de diverses façons avec les moyens bien connus de l'homme de l'art. On peut modifier les sites A1 ou A2 indépendamment ou simultanément.

La modification du site A2 peut consister en une modification, telle qu'une délétion totale, entraînant une perte d'antigénicité du site, mais on préfère que, comme pour le site A1, la modification exprime un épitope modifié de telle sorte que la protéine n'induise plus d'anticorps facilitants.

La région A1 a une partie commune avec la région dénommée "A2" dans la demande de brevet GB-A-2 282 601 (WO-A-95/07987) citée plus haut, mais contrairement à l'invention, celle-ci prévoit des mutations (modifications) ou des délétions entraînant une perte de l'antigénicité de la région modifiée ou délétée.

L'invention a donc en particulier pour objet une séquence nucléotidique comprenant le gène FIPV S complet, présentant au moins une modification, de préférence mutation et/ou délétion limitée, dans la région antigénique A2 qui code pour les acides aminés 637 à 662 et/ou dans la région antigénique A1 qui code pour les acides aminés 562 à 598, à l'exception, au moins pour le site A1, d'une délétion totale ou d'une mutation ou délétion importante ayant les mêmes conséquences qu'une délétion totale, à savoir une perte de l'antigénicité de la région modifiée.

La présente demande se trouve donc maintenant limitée à la partie de l'invention qui concerne les modifications permettant de supprimer l'induction d'anticorps facilitants sans supprimer l'antigénicité, au moins pour le site A1.

Bien entendu l'expression d'une séquence nucléotidique comprenant le gène FIPV S complet recouvre les souches FIPV types 1 et 2 ainsi que les variants et les séquences qui présentent des variations secondaires, c'est-à-dire qui ne touchent pas à l'immunogénicité de la protéine S, ce qui couvre aussi des mutations et délétions secondaires en dehors des sites A1 et A2. De préférence, les variations de la séquence ne doivent pas modifier la fonctionnalité de la glycoprotéine S.

Cela inclut donc les séquences possédant un degré d'homologie élevé avec les séquences précédentes, y compris en tenant compte de la dégénérescence du code génétique, cette homologie étant suffisamment élevée pour que le polypeptide exprimé permette d'induire une protection vaccinale efficace.

Par délétion limitée, on entend de préférence une délétion ponctuelle (correspondant à 1 acide aminé) ou une microdélétion (jusqu'à 6 acides aminés).

On évitera en général les mutations ou délétions des codons codant pour les cystéines situés dans A1 et A2.

On préférera en outre les mutations ponctuelles et en second lieu les délétions ponctuelles (sauf Cys) aux mutations et délétions plus étendues.

Pour le site A1, les modifications comprennent *a minima* une mutation pour l'un au moins et, de préférence, les deux codons codant pour Asp 568 et pour Asp 591 pour avoir tout autre acide aminé à ces positions. A la condition que les acides aminés 568 et 591 ne soient pas des Asp, tout autre acide aminé dans la région 562-598 peut être substitué à l'acide aminé naturel de la position considérée.

Les modifications du site A1 comprennent aussi les délétions limitées de cette région comprenant les acides aminés 568 et/ou 591.

Pour le site A2, les modifications comprennent *a minima* une mutation pour l'un au moins, et de préférence, les trois codons codant pour Asp 643, Arg 649 et Arg 656 pour avoir tout autre acide aminé à ces positions. Les modifications du site A2 comprennent aussi la délétion totale ou les délétions partielles de cette région comprenant les acides aminés 643, 649 et/ou 656.

La présente invention a encore pour objet l'utilisation des gènes FIPV ainsi modifiés pour l'expression *in vitro* de protéines FIPV S recombinantes et pour la préparation de vaccins sous-unités purifiées pour la vaccination des chats contre la PIF.

La présente invention a aussi pour objet l'utilisation des gènes FIPV S ainsi modifiés pour la construction de vecteurs viraux recombinants exprimant ces gènes modifiés. Ces vecteurs viraux peuvent être des virus recombinants réplicatifs ou non réplicatifs et plus particulièrement des poxvirus (exemple : virus de la vaccine et ses dérivés, canarypox virus...), des herpèsvirus (herpèsvirus félin en particulier), ou des adénovirus.

La présente invention a pour objet la préparation de vaccins contre la PIF avec ces virus recombinants.

La présente invention a encore pour objet l'immunisation de chats contre la PIF avec des plasmides contenant les gènes FIPV S, modifiés selon la présente invention, et placés sous le contrôle d'un promoteur fort (par exemple HCMV IE, SV40, etc.) et de signaux de régulation pour la transcription et la traduction. Les plasmides se trouvent dans un véhicule susceptible de permettre l'injection directe chez le chat, notamment par voie intramusculaire. Il s'agit notamment de plasmides nus tels que décrits dans la demande de brevet internationale WO 90/11092.

La présente invention a enfin pour objet la préparation de vaccins contre la PIF comprenant une (ou des) protéine(s) FIPV S, modifiée(s) selon la présente invention, de préférence associée(s) à d'autres protéines du virus FIPV telles que par exemple la protéine M.

Une autre solution vaccinale consiste à utiliser des cellules (en particulier d'origine féline) exprimant constitutivement la glycoprotéine S selon l'invention.

### Exemples :

### Exemple 1 : Clonage et expression des fragments du gène FIPV S.

Dans le but de localiser la région du gène FIPV S responsable pour la neutralisation et pour la facilitation, le clonage de fragments chevauchants du gène FIPV S a été entrepris de manière à exprimer ces fragments sous forme de protéines de fusion avec la protéine du gène 10 du phage T7. La séquence des oligonucléotides pour l'amplification des différents fragments a été choisie de manière à couvrir l'ensemble de la région codante du gène S en 3 grands fragments d'environ 1600 paires de bases (pb) et 12 sous-fragments plus petits d'environ 400 à 500 pb. Ces oligonucléotides contiennent les sites de restriction BamHI, XbaI ou XhoI pour faciliter leur clonage. La transcription réverse de l'ARN et l'amplification de l'ADN complémentaire par une réaction d'amplication en chaîne par polymérase ont été réalisées selon des techniques standards (Sambrook J. et al., Molecular Cloning : a laboratory manual, 2ème éd., Cold Spring Harbor Laboratory, Cold Spring Harbor, N.Y.). L'ADN amplifié a été digéré par les enzymes appropriées et cloné dans le vecteur pBluescript (Stratagene, La Jolla, Ca.).

Les limites des différents fragments clonés à partir du gène S de la souche 79-1146 du virus FIPV sont indiquées ci-dessous : (toutes les positions font référence à la séquence du gène S de la souche FIPV 79-1146 publiée par De Groot R. et al. (J. Gen. Virol. 1987, 68, 2639-2646).
Fragment F1 : nucléotides 70 à 1736.
Fragment F2 : nucléotides 1519 à 3160.
Fragment F3 : nucléotides 2773 à 4428.
Fragment S1 : nucléotides 70-535.
Fragment S2 : nucléotides 394-862.
Fragment S3 : nucléotides 742-1221.
Fragment S4 : nucléotides 1045-1539.
Fragment S5 : nucléotides 1339-1734.
Fragment S6 : nucléotides 1594-2089.
Fragment S7 : nucléotides 1963-2443.
Fragment S8 : nucléotides 2296-2838.
Fragment S9 : nucléotides 2743-3004.
Fragment S10 : nucléotides 2890-3506.
Fragment S11 : nucléotides 3352-4063.
Fragment S12 : nucléotides 3895-4428.

Les différents fragments FIPV clonés ont ensuite été isolés des clones Bluescript par digestion NotI et XhoI, puis reclonés dans le vecteur pTOPE-SX pour l'étape de transcription et de traduction *in vitro.*

La construction de pTOPE-SX est décrite ci-dessous :

Le plasmide pTOPE-1b(+) (Novagen) contient le promoteur T7 et une partie du gène 10 du phage T7 suivie d'un polylinker. Ce polylinker a été entièrement éliminé par digestion avec les enzymes de restriction SacII et XhoI et remplacé par le fragment SacII-XhoI de 82 pb isolé du polylinker contenu dans pBluescript. Un nucléotide supplémentaire a été ajouté à ce fragment de manière à mettre tous les fragments FIPV clonés dans pBluescript en phase avec la phase du gène 10. Le nouveau plasmide a été appelé pTOPE-SX. La transcrition et la traduction *in vitro* avec l'ARN polymérase du phage T7 des inserts contenus dans pTOPE-SX permet d'obtenir des protéines de fusion contenant 260 acides aminés de la protéine du gène 10 suivis des acides aminés codés par les inserts FIPV.

### Exemple 2 : Reconnaissance des peptides FIPV S par les anticorps monoclonaux.

Dans le but de localiser la région générale du gène S du virus FIPV responsable de la neutralisation et de la facilitation, des fragments chevauchants de ce gène ont été clonés par PCR dans le vecteur pBluescript sous la forme de trois grands fragments (F1, F2 et F3 ; figure 1) et de 12 petits sous-fragments (S1 à S12). Ces inserts FIPV ont ensuite été sous-clonés dans le vecteur pTOPE-SX en vue de leur transcription et de leur traduction *in vitro.*

Les réactions couplées de transcription et de traduction *in vitro* ont été réalisées en utilisant le système "TNT Reticulocyte Lysate" (Promega, Madison, WI) selon la technique recommandée par le fabricant en présence de ³⁵S-methionine (Amersham France). Pour étudier l'effet de la maturation post-traductionnelle des protéines , les réactions ont également été réalisées en présence de membranes microsomales pancréatiques de chien (Promega). Les produits de traduction ont été séparés par électrophorèse sur gel de polyacrylamide-SDS et révélés par autoradiographie.

Les essais de radio-immunoprecipitation (RIPA) ont été effectués en mélangeant 5*µ*l du mélange de traduction de la protéine de fusion avec 5*µ*l d'anticorps monoclonal ou de sérum de chat dans 200 *µ*l de tampon TNE Triton X-100 (NaCl 150 mM, Tris (pH 8,0) 50 mM, EDTA 5 mM, Triton X-100 0,1 %) et en agitant ce mélange à +4°c pendant 1 heure. Des sérums de chat positifs et négatifs pour FIPV ainsi qu'un monoclonal dirigé contre les 10 premiers acides aminés de la protéine du gène 10 de T7 (anticorps monoclonal T7 Tag, Novagen) ont été utilisés comme contrôles. Les immuns complexes sont adsorbés par addition de 50*µ*l d'un conjugué agarose-protéine G recombinante (Boehringer Mannheim, Mannheim, Germany) aux échantillons contenant les anticorps monoclonaux ou par addition de 50*µ*l d'un conjugué agarose-protéine A recombinante (Boehringer Mannheim) aux échantillons contenant les sérums de chats. Les immuns complexes fixés sur l'agarose ont été centrifugés pendant 30 s et lavés deux fois en tampon RIPA (150 mM NaCl, 50 mM Tris (pH 8,0), 1 % Triton X-100, 0,5 % sodium désoxycholate, 0,1 % SDS) et une fois avec le tampon Tris-Triton (10 mM Tris (pH 8,0), 0,1 % Triton X-100). Les échantillons centrifugés sont ensuite séparés par électrophorèse. Les gels sont fixés et traités avec une solution d'Amplify (Amersham) et révélés par autoradiographie.

Les grands fragments F1, F2 et F3 ont une taille d'environ 62 kDa, ce qui donnne des protéines de fusion d'environ 90 kDa correspondant effectivement aux tailles observées. Les petits fragments S1 à S12 ont des tailles d'environ 18 kDa, ce qui donne des protéines de fusion d'environ 46 kDa.

Pour optimiser les conditions de reconnaissance des peptides de fusion FIPV S par les anticorps monoclonaux, les protéines de fusion ont été également traduites en présence de membranes microsomales de pancréas de chien. La glycosylation de l'extrémité N-terminale de S (fragment F1) se traduit par un changement de la taille de la protéine de fusion F1 de 90 kDa à 98 kDa ou 145 kDa correspondant respectivement à un accroissement de 8 ou de 55 kDa. Une augmentation de 8 kDa est également observée pour la taille du sous-fragment S1 qui passe de 48 à 54 kDa. La taille des autres fragments FIPV S n'est pas modifiée par la traduction effectuée en présence de membranes microsomales.

Les anticorps monoclonaux spécifiques anti-FIPV S 23F4.5, 24H5.4 et 18A7.4 (Corapi W. et al., J. Virol. 1992, 66, 6695-6705) reconnaissent le fragment F2 et le sous-fragment S6 qui ont en commun une séquence de 165 acides aminés (positions 509 à 673 sur la séquence de la protéine S de la souche 79-1146 (De Groot R. et al., J. Gen. Virol. 1987, 68, 2639-2646). La reconnaissance du fragment F2 n'est pas améliorée par l'utilisation de protéines traduites en présence de membranes microsomales, ce qui suggère que la glycosylation n'est pas nécessaire pour la reconnaissance des épitopes recherchés.

### Exemple 3 : Séquençage des virus mutants résistants à la neutralisation par les anticorps monoclonaux anti-FIPV S (mutants "mar").

Pour localiser les sites antigéniques localisés sur le fragment S6, la région S6 de plusieurs mutants FIPV *mar* a été amplifiée par PCR et clonée dans le vecteur pBluescript SK+ et séquencée. La séquence des mutants *mar* a été établie sur des mutants *mar* indépendants obtenus avec le même monoclonal aussi bien qu'avec des clones issus d'amplifications PCR indépendantes avec le même mutant *mar.* La séquence de chaque clone a été établie sur les deux brins en utilisant le kit Sequenase (Amersham) selon la technique recommandée par le fabricant.

Les séquences obtenues ont été comparées avec la séquence homologue du virus parental 79-1146. Les mutants *mar* analysés sont les mutants identifiés *mar* 23F4.5, *mar* 18A7.4 et *mar* 24H5.4. Ces mutants ont été obtenus respectivement avec les anticorps monoclonaux 23F4.5, 18A7.4 et 24H5.4 décrits par C. Olsen (Olsen C. et al., J. Virology 1992, 66, 956-965) et W. Corapi (Corapi W. et al., J. Virology 1992, 66, 6695-6705).

Le monoclonal 23F4.5 possède un titre neutralisant de 20480 (Corapi, 1992) et induit une facilitation de l'infection *in vitro* d'au moins 100 fois le niveau normal (Olsen, 1992). Le mutant *mar* 23F4.5 présente des mutations aux positions 1840 et 2014 qui induisent des changements d'acides aminés dans la séquence de la protéine S pour les résidus 591 (Asp -> Tyr) et 649 (Arg -> Gly). Le monoclonal 18A7.4 possède un titre neutralisant de 5120 et induit une facilitation de l'infection *in vitro* d'au moins 100 fois le niveau normal. Le mutant *mar* 18A7.4 présente des mutations aux positions 1772 et 2036 qui induisent des changements d'acides aminés pour les résidus 568 (Asp -> Val) et 656 (Arg -> Lys).

Le monoclonal 24H5.4 possède un titre neutralisant de 96 et il a la particularité de ne pas induire de facilitation de l'infection (Olsen, 1992). Le mutant *mar* 24H5.4 présente une seule mutation à la position 1996 qui induit un changement d'acide aminé pour le résidu 643 (Asp -> Tyr).

### Exemple 4 : Mutagénèse du site A1.

Le fragment central du gène FIPV S HindIII-HindIII de 1723 pb (nucléotides 1696 à 3418) est cloné dans le vecteur pBS-SK+ pour donner le plasmide pFIPV-S2. Le site A1 est situé sur le sous-fragment HindIII-SspI (positions 1696 à 1845) de ce fragment. Le site A1 est mutagénisé par PCR en utilisant la stratégie suivante :
Les oligonucléotides suivants sont synthétisés :
   OLIGO A11 (95 mer) (SEQ ID NO: 1) =
   OLIGO A12 (88 mer) (SEQ ID NO: 2) =
   OLIGO A13 (20 mer) (SEQ ID NO: 3) =
   OLIGO A14 (20 mer) (SEQ ID NO: 4) =

Les oligonucléotides A11 et A12 sont hybridés entre eux grâce à leur séquence complémentaire commune de 23 paires de bases. L'hybride ainsi obtenu sert alors, après élongation de ses extrémités 3', de matrice pour une réaction PCR utlisant les oligonucléotides A13 et A14. Cette réaction d'amplification par PCR permet d'obtenir un fragment de 159 pb. Ce fragment est alors digéré avec les enzymes de restriction HindIII et SspI pour produire un fragment HindIII-SspI de 149 pb (fragment A). Ce fragment contient le site A1 modifié à deux positions (Val au lieu de Asp à la position 568 et Tyr au lieu de Asp à la position 591). Le plasmide pFIPV-S2 est digéré par HindIII et digéré partiellement par SspI pour isoler le fragment SspI-HindIII de 1569 pb (fragment B) par Geneclean (BI0101 Inc., La Jolla, Ca.). Le vecteur pBS-SK+ est digéré par HindIII et déphosphorylé pour produire le fragment C (2960 pb).

Les fragments A, B et C sont alors ligaturés ensemble pour produire le plasmide pFIPSA1*. Ce plasmide contient le fragment HindIII-HindIII du gène FIPV S modifié pour deux acides aminés du site A1.

Le gène FIPV S est ensuite reconstitué en remplaçant par simple clonage le fragment HindIII-HindIII naturel (positions 1696 à 3418) par le fragment HindIII-HindIII contenu dans le plasmide pFIPSA1*. Le gène complet FIPV S modifié au site A1 peut alors être utilisé pour les constructions de plasmides d'expression ou de virus recombinants.

### Exemple 5 : Hutagénèse du site A2.

Les oligonucléotides suivants sont synthétisés :
OLIGO A21 (20 mer) (SEQ ID NO: 5) =
OLIGO A22 (36 mer) (SEQ ID NO: 6)
OLIGO A23 (36 mer) (SEQ ID NO: 7) =
OLIGO A24 (20 mer) (SEQ ID NO: 8) =

Une réaction PCR (PCR A) est réalisée avec les oligonucléotides A21 et A22 et avec le plasmide pFIPV-S2 comme matrice pour synthétiser un fragment PCR de 199 pb (fragment A).

Une réaction PCR (PCR B) est réalisée avec les oligonucléotides A23 et A24 et avec le plasmide pFIPV-S2 comme matrice pour donner un fragment PCR de 273 pb (fragment B).

Les fragments PCR A et B sont hybridés entre eux grâce à leur région complémentaire de 46 pb et le produit de cette hybridation, après élongation des extrémités 3', est amplifié par une réaction PCR (PCR C) avec les oligonucléotides A21 et A24 pour donner un fragment PCR de 424 pb. Ce fragment PCR est alors digéré par SspI et DraI pour donner le fragment de restriction SspI-DraI de 402 pb (fragment C).

Le plasmide pFIPV-S2 est digéré par HindIII et SspI pour isoler le fragment HindIII-SspI de 149 pb (fragment D).

Le plasmide pFIPV-S2 est digéré par HindIII et DraI pour isoler le fragment de restriction DraI-HindIII de 1170 pb (fragment E). Le vecteur pBS-SK+ est digéré par HindIII et déphosphorylé pour donner le fragment F (2960 pb).

Les fragments C, D, E et F sont ligaturés ensemble pour donner le plasmide pFIPSA2*. Le fragment central HindIII-HindIII 1723 pb du gène FIPV S contenu dans pFIPSA2* possède un site A2 modifié au niveau de 3 acides aminés (Tyr au lieu de Asp à la position 643, Gly au lieu de Arg à la position 649, et Lys au lieu de Arg à la position 656).

Le gène FIPV S est ensuite reconstitué en remplaçant par simple clonage le fragment HindIII-HindIII naturel (positions 1696 à 3418) par le fragment HindIII-HindIII contenu dans le plasmide pFIPSA2*. Le gène complet FIPV S modifié au site A2 peut alors être utilisé pour les constructions de plasmides d'expression ou de virus recombinants.

### Exemple 6 : Mutagénèse des sites A1 et A2.

Les fragments A (exemple 4), C et E (exemple 5) sont ligaturés avec le vecteur pBS-SK+, préalablement digéré par HindIII et déphosphorylé, pour donner le plasmide pFIPSA1*A2*. Le fragment central HindIII-HindIII 1723 pb du gène FIPV S contenu dans pFIPSA1*A2* présente 2 changements d'acides aminés au niveau du site A1 (voir exemple 4) et 3 changements d'acides aminés au niveau du site A2 (voir exemple 5).

Le gène FIPV S est ensuite reconstitué en remplaçant par simple clonage le fragment HindIII-HindIII naturel par le fragment HindIII-HindIII 1723 pb contenu dans pFIPSAl*A2*. Le gène complet FIPV S comprenant des modifications au niveau des sites A1 et A2 peut alors être utilisé pour la construction de plasmides d'expression ou de virus recombinants.

### Exemple 7 : Construction de délétions au niveau des sites A1 et A2.

Selon la stratégie de clonage décrite précédemment (mutagénèse par utilisation de réactions PCR), des délétions respectant la phase de lecture du gène FIPV S peuvent être introduites au niveau des sites A1 et/ou A2. Selon le même schéma que décrit précédemment (voir exemple 6), on peut construire un fragment central HindIII-HindIII du gène FIPV S présentant une délétion dans le site A1 et/ou une délétion dans le site A2.

## Revendications

1. Un acide nucléique codant pour une protéine S du virus de la péritonite infectieuse féline (FIPV), modifiée pour ne pas induire d'anticorps facilitants, comprenant :
(a) au moins une délétion partielle comprenant les acides aminés Asp 568 et/ou Asp 591 de la région antigénique A1 de la protéine S, région délimitée par les acides aminés Met 562 à Cys 598 et au moins une modification dans les nucléotides codant pour la région antigénique A2 de la protéine S, région délimitée par les acides aminés Gly 637 à Tyr 662,ou
(b) une mutation pour l'un au moins et, de préférence, les deux codons codant pour Asp 568 et pour Asp 591 de ladite région antigénique A1 pour avoir tout autre acide aminé à cette position et au moins une modification dans les nucléotides codant pour ladite région antigénique A2, ou
(c) au moins une mutation pour tout acide aminé de ladite région antigénique A1, à la condition que les acides aminés Asp 568 et Asp 591 ne soient plus des Asp, et au moins une modification dans les nucléotides codant pour ladite région antigénique A2, ou
(d) une délétion partielle de la région antigénique A1 comprenant l'acide aminé Asp 568, ou
(e) une délétion partielle de la région antigénique A1 comprenant les acides aminés Asp 568 et Asp 591, ou
(f) une mutation pour le codon codant pour Asp 568 pour avoir tout autre acide aminé à cette position et de préférence pour les deux codons codant pour Asp 568 et pour Asp 591 de ladite région antigénique A1, ou
(g) au moins une mutation pour tout acide aminé de ladite région antigénique A1, à la condition que les acides aminés Asp 568 et Asp 591 ne soient plus des Asp, ou
(h) au moins une modification dans les nucléotides codant pour ladite région antigénique A2,
la modification de la région antigénique A2 prévue en a), b), c) ou h) comprenant soit a minima une mutation pour l'un au moins, et de préférence, les trois codons pour Asp 643, Arg 649 et Arg 656 pour avoir tout autre acide aminé à ces positions, soit une délétion partielle comprenant les acides aminés Asp 643, Arg 649 et/ou Arg 656.

2. L'acide nucléique de la revendication 1, dans laquelle les nucléotides codant pour la région antigénique A2 sont délétés.

3. L'acide nucléique de la revendication 1 ou 2, comprenant une mutation dans au moins l'un des codons codant pour Asp 568 et Asp 591, le codon muté codant pour un autre acide aminé.

4. L'acide nucléique selon l'une quelconque des revendications 1 à 3, comprenant une délétion d'au moins l'un des codons codant pour Asp 568 et Asp 591.

5. L'acide nucléique selon l'une quelconque des revendications 1 à 3, comprenant au moins une délétion incluant au moins l'un des codons codant pour Asp 568 et Asp 591.

6. L'acide nucléique selon l'une quelconque des revendications 1 ou 3 à 5, comprenant une mutation dans l'un au moins des codons codant pour Asp 643, Arg 649 et Arg 656, le codon muté codant pour un autre acide aminé.

7. L'acide nucléique selon l'une quelconque des revendications 1 ou 3 à 5, comprenant une délétion d'au moins l'un des codons codant pour Asp 643, Arg 649 et Arg 656.

8. L'acide nucléique selon l'une quelconque des revendications 1 ou 3 à 5, comprenant au moins une délétion incluant au moins un des codons codant pour Asp 643, Arg 649 et Arg 656.

9. Un polypeptide codé par une séquence nucléotidique selon l'une quelconque des revendications 1 à 8.

10. Un virus recombinant ou un plasmide comprenant une séquence nucléotidique codant et exprimant le polypeptide de la revendication 9.

11. Un vaccin comprenant un virus recombinant contenant et exprimant une séquence nucléotidique selon l'une quelconque des revendications 1 à 8, pour la vaccination d'un chat contre FIPV.

12. Le vaccin selon la revendication 11, dans lequel le virus recombinant est un poxvirus, herpesvirus ou adénovirus recombinant.

13. Le vaccin selon la revendication 12, dans lequel le poxvirus recombinant est un virus de la vaccine recombinant ou un virus canarypox recombinant.

14. Le vaccin selon la revendication 12, dans lequel l'herpesvirus recombinant est un herpesvirus félin recombinant.

15. Un vaccin comprenant un plasmide contenant et exprimant une séquence nucléotidique selon l'une quelconque des revendications 1 à 8, dans un véhicule susceptible de permettre l'injection directe du plasmide chez le chat, pour la vaccination d'un chat contre FIPV.

16. Le vaccin de la revendication 15, dans lequel le plasmide comprend ladite séquence nucléotidique sous le contrôle du promoteur HCMV IE et/ou d'un promoteur SV40.

17. Un vaccin comprenant un polypeptide selon la revendication 9.

18. Le vaccin de la revendication 17, comprenant en outre une autre protéine de FIPV.

19. Le vaccin de la revendication 17, comprenant en outre la protéine M de FIPV.

## Patentansprüche

1. Nucleinsäure, die für ein S-Protein des Virus der infektiösen Katzenperitonitis (FIPV) codiert, das modifiziert worden ist, um keine begünstigenden Antikörper zu induzieren, und welche:
a) mindestens eine partielle Deletion, welche die Aminosäuren Asp 568 und/oder Asp 591 der Antigenregion A1 des S-Proteins umfasst, wobei die Region von den Aminosäuren Met 562 bis Cys 598 begrenzt wird, und mindestens eine Modifizierung in den Nucleotiden, die für die Antigenregion A2 des S-Proteins codieren, wobei die Region von den Aminosäuren Gly 637 bis Tyr 662 begrenzt wird,
b) eine Mutation von wenigstens einem und vorzugsweise den zwei Codons, die für Asp 568 und für Asp 591 der Antigenregion A1 codieren, um eine beliebige andere Aminosäure in dieser Position zu haben, und mindestens eine Modifizierung in den Nucleotiden, die für die Antigenregion A2 codieren,
c) mindestens eine Mutation für jede Aminosäure der Antigenregion A1 unter der Bedingung, dass die Aminosäuren Asp 568 und Asp 591 keine Asp mehr sind, und mindestens eine Modifizierung in den Nucleotiden, die für die Antigenregion A2 codieren,
d) eine partielle Deletion der Antigenregion A1, welche die Aminosäure Asp568 umfasst,
e) eine partielle Deletion der Antigenregion A1, welche die Aminosäuren Asp 568 und Asp 591 umfasst,
f) eine Mutation für das Codon, das für Asp 568 codiert, um eine beliebige andere Aminosäure in dieser Position zu haben, und vorzugsweise für die zwei Codons, die für Asp 568 und für Asp 591 der Antigenregion A 1 codieren,
g) mindestens eine Mutation für jede Aminosäure der Antigenregion A1 unter der Bedingung, dass die Aminosäuren Asp 568 und Asp 591 keine Asp mehr sind, oder
h) mindestens eine Modifizierung in den Nucleotiden, die für die Antigenregion A2 codieren,
umfasst, wobei die Modifizierung der Antigenregion A2, die in a), b), c) oder h) vorgesehen ist, entweder wenigstens eine Mutation für mindestens eines und vorzugsweise die drei Codons für Asp 643, Arg 649 und Arg 656, um eine beliebige andere Aminosäure in diesen Positionen zu haben, oder eine partielle Deletion, welche die Aminosäuren Asp 643, Arg 649 und/oder Arg 656 umfasst, umfasst.

2. Nucleinsäure nach Anspruch 1, in welcher die für die Antigenregion A2 codierenden Nucleotide deletiert sind.

3. Nucleinsäure nach Anspruch 1 oder 2, die eine Mutation in mindestens einem der für Asp 568 und Asp 591 codierenden Codons enthält, wobei das mutierte Codon für eine andere Aminosäure codiert.

4. Nucleinsäure nach einem der Ansprüche 1 bis 3, die eine Deletion von mindestens einem der Codons, die für Asp 568 und Asp 591 codieren, enthält.

5. Nucleinsäure nach einem der Ansprüche 1 bis 3, die mindestens eine Deletion enthält, die wenigstens eines der Codons enthält, die für Asp 568 und Asp 591 codieren.

6. Nucleinsäure nach einem der Ansprüche 1 oder 3 bis 5, die eine Mutation in mindestens einem der Codons enthält, die für Asp 643, Arg 649 und Arg 656 codieren, wobei das mutierte Codon für eine andere Aminosäure codiert.

7. Nucleinsäure nach einem der Ansprüche 1 oder 3 bis 5, die eine Deletion von mindestens einem der Codons, die für Asp 643, Arg 649 und Arg 656 codieren, enthält.

8. Nucleinsäure nach einem der Ansprüche 1 oder 3 bis 5, die wenigstens eine Deletion enthält, die mindestens eines der Codons einschließt, die für Asp 643, Arg 649 und Arg 656 codieren.

9. Polypeptid, für welches eine Nucleotidsequenz nach einem der Ansprüche 1 bis 8 codiert.

10. Rekombinantes Virus oder ein Plasmid, das eine Nucleotidsequenz enthält, die das Polypeptid des Anspruchs 9 codiert und exprimiert.

11. Impfstoff für die Impfung einer Katze gegen FIPV, der ein rekombinantes Virus umfasst, das eine Nucleotidsequenz nach einem der Ansprüche 1 bis 8 enthält und exprimiert.

12. Impfstoff nach Anspruch 11, in welchem das rekombinante Virus ein rekombinantes Pockenvirus, Herpesvirus oder Adenovirus ist.

13. Impfstoff nach Anspruch 12, in welchem das rekombinante Pockenvirus ein rekombinantes Virus des Impfstoffs oder ein rekombinantes Canarypoxvirus ist.

14. Impfstoff nach Anspruch 12, in welchem das rekombinante Herpesvirus ein rekombinantes Katzenherpesvirus ist.

15. Impfstoff, der ein Plasmid umfasst, das eine Nucleotidsequenz nach einem der Ansprüche 1 bis 8 enthält und exprimiert, in einem Träger, der in der Lage ist, eine direkte Injektion des Plasmids in eine Katze zu erlauben, um diese gegen FIPV zu impfen.

16. Impfstoff nach Anspruch 15, in welchem das Plasmid die Nucleotidsequenz unter der Kontrolle des Promotors HCMV IE und/oder eines Promotors SV40 enthält.

17. Impfstoff, der ein Polypeptid nach Anspruch 9 umfasst.

18. Impfstoff nach Anspruch 17, der außerdem ein anderes FIPV-Protein enthält.

19. Impfstoff nach Anspruch 17, der darüber hinaus das FIPV-M-Protein enthält.

## Claims

1. A nucleic acid coding for an S protein of the feline infectious peritonitis virus (FIPV) modified so that it does not induce facilitating antibodies, including:
a) at least one partial deletion including amino acids Asp568 and/or Asp591 from the antigenic region A1 of the S protein, a region delimited by amino acids Met562 to Cys598, and at least one modification in the nucleotides coding for the antigenic region A2 of the S protein, a region delimited by amino acids Gly637 to Tyr662; or
b) a mutation for at least one and preferably the two codons coding for Asp568 and for Asp591 of said antigenic region A1 to have any other amino acid in said position, and at least one modification in the nucleotides coding for said antigenic region A2; or
c) at least one mutation for any amino acid of said antigenic region A1, provided that amino acids Asp568 and Asp591 are no longer Asps, and at least one modification in the nucleotides coding for said antigenic region A2; or
d) a partial deletion from the antigenic region A1 including amino acid Asp568; or
e) a partial deletion from the antigenic region A1 including amino acids Asp568 and Asp591 ; or
f) a mutation for the codon coding for Asp568 to have any other amino acid in said position, and preferably for the two codons coding for Asp568 and for Asp591 of said antigenic region A1 ; or
g) at least one mutation for any amino acid of said antigenic region A1, provided that amino acids Asp568 and Asp 591 are no longer Asp ; or
h) at least one modification en the nucloeotides coding for said antigenic region A2 ; the modification in the antigenic region A2 envisaged in a), b), c), or h) including either a minimum of one mutation for at least one and preferably the three codons for Asp643, Arg649 and Arg656 to have any other amino acid at said positions, or a partial deletion including amino acids Asp63, Arg649 abd/or Arg656.

2. The nucleic acid of claim 1, wherein the nucleotides coding for the antigenic region A2 are deleted.

3. The nucleic acid of claim 1 or claim 2, including a mutation in at least one of the codons coding for Asp568 and Asp591, the mutated codon coding for another amino acid.

4. The nucleic acid according to any one of claims 1 to 3, including a deletion of at least one of the codons coding for Asp568 and Asp591.

5. The nucleic acid according to any one of claims 1 to 3, including at least one deletion including at least one of the codons coding for Asp568 and Asp591.

6. The nucleic acid according to any one of claims 1 or 3 to 5, including a mutation in at least one of the codons coding for Asp643, Arg649 and Arg656, the mutated codon coding for another amino acid.

7. The nucleic acid according to any one of claims 1 or 3 to 5, including a deletion of at least one of the codons coding for Asp643, Arg649 and Arg656.

8. The nucleic acid according to any one of claims 1 or 3 to 5, including at least one deletion including at least one of the codons coding for Asp643, Arg649 and Arg656.

9. A polypeptide coded by a nucleotide sequence according to any one of claims I to 8.

10. A recombinant virus or a plasmid including a nucleotide sequence coding for and expressing the polypeptide of claim 9.

11. A vaccine including a recombinant virus containing and expressing a nucleotide sequence according to any one of claims 1 to 8, for vaccination of a cat against FIPV.

12. The vaccine according to claim 11, wherein the recombinant virus is a recombinant pox virus, herpes virus or adenovirus.

13. The vaccine according to claim 12, wherein the recombinant pox virus is a recombinant vaccine virus or a recombinant canary pox virus.

14. The vaccine according to claim 12, in which the recombinant herpes virus is a recombinant feline herpes virus.

15. A vaccine including a plasmid containing and expressing a nucleotide sequence according to any one of claims 1 to 8, in a vehicle which allows direct injection of the plasmid into a cat, to vaccinate a cat against FIPV.

16. The vaccine of claim 15, in which the plasmid includes said nucleotide sequence under the control of the HCMV 1E promoter and/or a SV40 promoter.

17. A vaccine including a polypeptide according to claim 9.

18. The vaccine of claim 17, further including a further FIPV protein.

19. The vaccine of claim 17, further including the M protein of FIPV.


### SEQUENCE LISTING

<110> MERIAL
<120> Infectious peritonitis vaccine
<130> FIPV
<140> EP 95 929 143.6
   <141> 1995-08-28
<150> FR 94/103739
   <151> 1994-08-29
<160> 8
<170> PatentIn version 3.2
<210> 1
   <211> 95
   <212> DNA
   <213> Artificial
<220>
   <223> oligonucleotide as PCR primer
<400> 1
   atgaagctta gtggttatgg tcaacccata gcctcgacac taagtaacat cacactacca 60
   atgcaggata acaatactgt tgtgtactgt attcg 95
<210> 2
   <211> 88
   <212> DNA
   <213> Artificial
<220>
   <223> oligonucleotide as PCR primer
<400> 2
   aaaaatattg taccataaag aacttttgca agtggaatga acataaactg agaattggtt 60
   agaacgaata cagtacacaa cagtattg 88
<210> 3
   <211> 20
   <212> DNA
   <213> Artificial
<220>
   <223> Oligonucleotide as PCR primer
<400> 3
   atgaagctta gtggttatgg 20
<210> 4
   <211> 20
   <212> DNA
   <213> Artificial
<220>
   <223> Oligonucleotide as PCR primer
<400> 4
   aaaaatattg taccataaag 20
<210> 5
   <211> 20
   <212> DNA
   <213> Artificial
<220>
   <223> oligonucleotide as PCR primer
<400> 5
   ggacaatatt tttaatcaag 20
<210> 6
   <211> 36
   <212> DNA
   <213> Artificial
<220>
   <223> oligonucleotide as PCR primer
<400> 6
   tttaacaacc tgctcattgg ttcctgtacg tgcagc 36
<210> 7
   <211> 36
   <212> DNA
   <213> Artificial
<220>
   <223> oligonucleotide as PCR primer
<400> 7
   aagttttatg ttgctgcacg tacaggaacc aatgag 36
<210> 8
   <211> 20
   <212> DNA
   <213> Artificial
<220>
   <223> oligonucleotide as PCR primer
<400> 8
   atcactaaca tttttaaagc 20